# EUROPÄISCHE PATENTSCHRIFT

(11) **EP 0 395 089 B1**
(45) Veröffentlichungstag und Bekanntmachung des Hinweises auf die Patenterteilung: **06.07.1994**
(21) Anmeldenummer: 90108069.7
(22) Anmeldetag: 27.04.1990
(51) Int. Cl.: A61B 5/103

(54) **Parallelführungseinrichtung für einen Ohrtaster**
Ear probe provided with a parallel guiding device
Dispositif de guidage parallèle pourvu d'un étrier contactant l'oreille

(30) Priorität: 28.04.1989 DE 3914137
(43) Veröffentlichungstag der Anmeldung: 31.10.1990
(73) Patentinhaber: EYEMETRICS-SYSTEMS AG, CH-7000 Chur (CH)
(72) Erfinder: Lange, Karl Heinz, D-4980 Bünde (DE)
(74) Vertreter: Grams, Klaus Dieter, Dipl.-Ing.

(56) Entgegenhaltungen:
- EP-A- 0 237 687
- AU-B- 527 289
- DE-A- 3 545 875

## Beschreibung

Die Erfindung bezieht sich auf eine Parallelführungseinrichtung für einen Ohrtaster gemäß dem Oberbegriff von Patentanspruch 1.

Eine solche Parallelführungseinrichtung ist bekannt (DE-OS 35 45 875). Die bekannte Parallelführungseinrichtung kommt zur Anwendung bei einer Vorrichtung zum fotogrammetrischen Erfassen des menschlichen Kopfes. Der bekannte Ohrtaster besteht aus einem länglichen, im wesentlichen horizontal verlaufenden Abschnitt sowie einem von diesem länglichen Abschnitt im wesentlichen nach unten verlaufenden, gekrümmten Anlageabschnitt, der am Kopf in die Rille zwischen Ohr und Schädel eingelegt wird. Die Parallelführungseinrichtung dient dazu, den Ohrtaster relativ zur Vorrichtung derart vertikal und horizontal parallel zu sich selbst verlagern zu können, daß das vorstehend beschriebene Anlegen des Anlageabschnitts je nach der individuellen Geometrie des Kopfes und Ohres erfolgen kann. Der Ohrtaster ist mit Marken versehen, die fotogrammetrisch mittels der Vorrichtung erfaßbar sind, so daß aus den so gewonnenen Daten auf die Geometrie des Bereichs hinter dem jeweiligen Ohr bzw. zwischen dem Ohr und dem Schädel geschlossen werden kann.

Damit der Ohrtaster beim Anlegen auch um die Längsachse des länglichen Abschnitts gedreht werden kann und auf diese Weise die von Individuum zu Individuum unterschiedliche Innenneigung der Rille zwischen Ohr und Schädel erfaßt werden kann, sind bei der bekannten Parallelführungseinrichtung die Gelenke der zweiten Parallelogrammführung vorzugsweise als Kugelgelenke ausgebildet, so daß die zweite Parallelogrammführung etwas verdreht werden kann. Aufgrund der vorstehend beschriebenen Ausbildung der bekannten Parallelführungseinrichtung kann es dazu kommen, daß der Ohrtaster und somit die von ihm getragenen Marken nicht nur stets parallel zu sich verlagert werden, sondern in geringem Maße auch gekippt oder geneigt werden. Dies kann die Meßgenauigkeit beeinträchtigen.

Der Erfindung liegt die Aufgabe zugrunde, die gattungsgemäße Parallelführungseinrichtung dahingehend zu verbessern, daß sie den Ohrtaster mit höherer Genauigkeit parallel führt, ohne daß die Möglichkeit verlorengeht, die Innenneigung des Anlageabschnitts des Ohrtasters verändern bzw. einstellen zu können.

Diese Aufgabe wird erfindungsgemäß gelöst durch die Merkmale im kennzeichnenden Teil von Patentanspruch 1.

Wesentlich für die Erfindung ist, daß alle vier Gelenke der ersten Parallelogrammführung einachsige Gelenke sind, d.h. eine Bewegung um lediglich eine Gelenkachse ermöglichen, daß die Gelenke der zweiten Lenker am bewegbaren Kopplungsglied jeweils zwei in bestimmter Weise verlaufende Gelenkachsen - und zwar nur zwei Gelenkachsen - aufweisen und daß für die gewünschte Einstellung der Innenneigung des Ohrtasters, d.h. die gewünschte Drehbarkeit des Ohrtasters, ein zusätzliches Gelenk mit in bestimmter Richtung verlaufender Gelenkachse vorgesehen ist.

Durch die erfindungsgemäße Ausbildung ist dafür gesorgt, daß die beiden Parallelogrammführungen nicht verdreht werden können und für genaue Parallelführung des weiteren Kopplungsgliedes sorgen. Dies bewirkt die genaue Parallelführung des Ohrtasters und der von ihm getragenen Marken. Zugleich ist jedoch die Möglichkeit gewährleistet, den Ohrtaster um die Längsachse seines länglichen Abschnitts zu drehen und außerdem in einer zweiten, zur ersten horizontalen Bewegungsrichtung des Ohrtasters senkrechten Horizontalrichtung verlagern zu können, um eine Anpassungsmöglichkeit an unterschiedliche Kopfbreiten zu bieten, die bei der bekannten Parallelführungseinrichtung durch deren Kugelgelenke gegeben ist.

Vorteilhafte Ausgestaltungen der Erfindung sind in den Unteransprüchen gekennzeichnet.

Von besonderem Vorteil sind die Maßnahmen gemäß Patentanspruch 10. Durch diese ist gewährleistet, daß von der ersten Parallelogrammführung auf die zweite Parallelogrammführung und somit auf den Ohrtaster eine horizontale Zugkraft ausgeübt wird, die unabhängig von dem Winkel, den die ersten Lenker mit der Senkrechten einschließen, und konstant ist.

Ein Ausführungsbeispiel der Erfindung ist in Zeichnungen dargestellt und wird im folgenden näher erläutert. Es zeigen:
Fig. 1 eine schematische Darstellung einer Vorrichtung zum fotogrammetrischen Erfassen des menschlichen Kopfes, bei der die erfindungsgemäße Parallelführungseinrichtung angewendet ist;
Fig. 2 eine perspektivische Ansicht eines Abschnitts einer Ausführungsform der erfindungsgemäßen Parallelführungseinrichtung;
Fig. 3 einen Horizontalschnitt gemäß A-A in Fig. 2 durch einen Ohrtaster und dessen Verbindungsvorrichtung; und
Fig. 4 eine Schnittdarstellung des Ohrtasters gemäß B-B in Fig. 3.

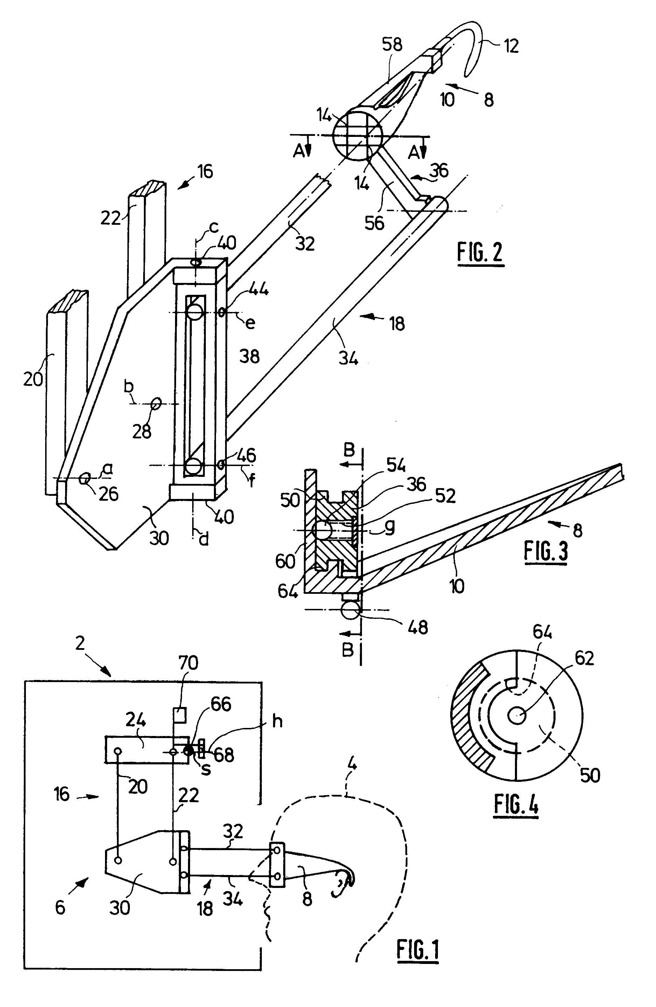

Fig. 1 zeigt schematisch und lediglich durch die Umrisse ihres Gehäuses angedeutet eine Vorrichtung 2 zum fotogrammetrischen Erfassen eines menschlichen Kopfes 4. Diese Vorrichtung 2 sowie ihre Arbeitsweise werden hier nicht erläutert. Ausführlich beschrieben ist sie in der DE-OS 35 45 875.

An der Vorrichtung 2 sind zwei Parallelführungseinrichtungen 6 befestigt, von denen lediglich eine in Fig. 1 gezeigt ist. Jede der beiden Parallelführungseinrichtungen 6 dient dazu, einen Ohrtaster 8 derart verlagerbar zu halten und zu führen, daß er in senkrechter Richtung, d.h. senkrecht in der Zeichenebene von Fig. 1, in einer ersten Horizontalrichtung, d.h. waagerecht in der Zeichenebene von Fig. 1, und außerdem in einer zweiten Horizontalrichtung, nämlich senkrecht zur Zeichenebene von Fig. 1 parallel zu sich selbst verlagert werden kann. Außerdem ist der Ohrtaster 8 um seine eigene Längsachse drehbar, die im wesentlichen horizontal in der Zeichenebene von Fig. 1 verläuft. Der Ohrtaster 8 umfaßt einen länglichen, im wesentlichen waagerechten Abschnitt 10, an dessen freiem, in Fig. 1 rechtem Ende ein Anlageabschnitt 12 angesteckt ist. Der Anlageabschnitt 12 wird in Anlage an der Rille zwischen dem Ohr und dem Schädel des Kopfes 4 auf der Rückseite des Ohres gebracht. Die vorstehend erläuterten, durch die Parallelführungseinrichtung gegebenen Bewegungsfreiheitsgrade dienen dazu, das Anlegen des Anlageabschnitts 12 des Ohrtasters 8 zu ermöglichen. Der Ohrtaster 8 ist auf seiner Vorderseite mit Marken 14 (siehe Fig. 2) versehen, deren Orte im Raum bei der fotogrammetrischen Erfassung gemessen werden, so daß aus den Orten der Marken 14 auf die dreidimensionale Geometrie des Bereichs hinter dem Ohr geschlossen werden kann.

Die Parallelführungseinrichtung 6 umfaßt eine erste Parallelogrammführung 16 sowie eine zweite Parallelogrammführung 18.

Die erste Parallelogrammführung 16 umfaßt zwei gleich lange, parallel zueinander verlaufende erste Lenker 20 und 22, die an ihren in Fig. 1 oberen Enden jeweils mittels eines einachsigen Gelenkes schwenkbar an einem vorrichtungsfesten Kopplungsglied 24 angelenkt sind. An ihren unteren Enden sind die beiden ersten Lenker jeweils mittels eines einachsigen Gelenks 26 bzw. 28 an einem bewegbaren Kopplungsglied 30 schwenkbar angelenkt. Die vier Gelenke der ersten Parallelogrammführung 16 können als Zapfenlager ausgebildet sein. Ihre vier Gelenkachsen, von denen die Gelenkachsen a und b der Gelenke 26 und 28 in Fig. 2 angedeutet sind, verlaufen parallel zueinander. Die Bewegungsebene, in der sich die beiden ersten Lenker 20 und 22 bewegen können, verläuft im wesentlichen senkrecht, d.h. in der Zeichenebene von Fig. 1. Da die vier Gelenke der ersten Parallelogrammführung 16 jeweils einachsige Gelenke sind, d.h. Gelenke mit nur einer Gelenkachse, ist die erste Parallelogrammführung 16 verwindungssteif.

Die zweite Parallelogrammführung 18 umfaßt zwei gleich lange, parallel zueinander verlaufende zweite Lenker 32 und 34. Diese zweiten Lenker 32 und 34 sind an ihren einen Enden schwenkbar am bewegbaren Kopplungsglied 30 angelenkt und an ihren anderen Enden gelenkig mit einem weiteren Kopplungsglied 36 verbunden.

Die gelenkige Verbindung zwischen den zweiten Lenkern 32 und 34 und dem als im wesentlichen dreieckige Platte ausgebildeten bewegbaren Kopplungsglied 30 andererseits ist auf folgende Weise ausgebildet. Am Kopplungsglied 30 ist ein Teil 38 in Form eines länglichen Rahmens mit Hilfe eines oberen Zapfenlagers 40 und eines unteren Zapfenlagers 42 drehbar gelagert. Die Gelenkachsen c und d der Zapfenlager 40 und 42 fluchten miteinander und verlaufen parallel zur Bewegungsebene der ersten Lenker 20 und 22. Die einen Enden der zweiten Lenker 32 und 34 verlaufen durch die Öffnung des rahmenförmigen Teils 38 und sind an diesem jeweils mittels eines einachsigen Gelenks 44 bzw. 46, das als Zapfenlager ausgebildet ist, schwenkbar gelagert. Die Gelenkachsen e und f der Gelenke 44 und 46 verlaufen parallel zueinander und senkrecht zu den Gelenkachsen c und d. Auf diese Weise sind die beiden zweiten Lenker 32 und 34 am bewegbaren Kopplungsglied 30 jeweils um zwei Gelenkachsen - und nur um zwei Gelenkachsen - schwenkbar, nämlich einerseits die Gelenkachsen e und f und andererseits die dazu senkrechten, miteinander fluchtenden Gelenkachsen c und d. Die beiden zweiten Lenker 32 und 34 können sich auf diese Weise in einer senkrechten Bewegungsebene bewegen, wobei diese Bewegungsebene der zweiten Lenker im wesentlichen parallel zur Bewegungsebene der ersten Lenker 20 und 22 verläuft, jedoch wegen der Drehbarkeit um die Gelenkachsen c und d relativ zur Bewegungsebene der ersten Lenker 20 und 22 aus der Parallellage heraus verschwenkbar ist. In sich ist die zweite Parallelogrammführung 18 wegen der beschriebenen Anlenkung am bewegbaren Kopplungsglied 30 gegen Verdrehen bzw. Verdrillen steif.

Die nicht näher dargestellten Gelenke zwischen den in den Fig. 1 und 2 rechten Enden der zweiten Lenker 32 und 34 einerseits und dem weiteren Kopplungsglied 36 andererseits sind als Kugelgelenke ausgebildet. Von diesen ist lediglich eine Kugel 48 des unteren Kugelgelenks in Fig. 3 erkennbar.

Das weitere Kopplungsglied 36 hat die Form eines weit geöffneten "V" und ist mit einer kreiszylindrischen Scheibe 50 versehen, deren Zylinderachse g mit der Längsachse des Ohrtasters 8 zusammenfällt und im wesentlichen horizontal in der Zeichenebene von Fig. 1 verläuft. Die Stirnseite der Scheibe 50 ist dem beweglichen Kopplungsglied 30 zugewandt und weist somit weg vom Anlageabschnitt 12. Entlang der Achse g ist im weiteren Kopplungsglied 36 eine Bohrung 52 ausgebildet, in der eine federbelastete Kugel 54 sitzt, die über die Stirnseite der Scheibe 50 nach links (in Fig. 3) vorsteht und entgegen der Federbelastung nach rechts in Fig. 3 gedrückt werden kann. Die kreiszylindrische Scheibe 50 befindet sich ungefähr an der Basis des "V" und steht von der durch die Schenkel 56 und 58 des weiteren Kopplungsgliedes 36 gebildeten Ebene in Richtung zum bewegbaren Kopplungsglied 30 vor.

Am vorderen, in Fig 3 linken Ende des Ohrtasters 8 ist eine zur Scheibe 50 parallele und konzentrische Scheibe 60 angeformt. Diese Scheibe trägt auf ihrer Vorderseite die Marken 14. Auf ihrer Rückseite ist die Scheibe 60 mit einer Ausnehmung 62 versehen, in die die Kugel 54 einrasten kann. Auf der Rückseite der Scheibe 60 ist am Ohrtaster 8 eine Ringnut 64 ausgebildet, in die der Rand der Scheibe 50 eingesetzt ist, so daß er von der Ringnut 64 umgriffen wird. Allerdings erstreckt sich die Ringnut 64 nur über einen Abschnitt des Umfangs der Scheibe 50, und zwar vorzugsweise von etwas weniger als 180°, wie Fig. 4 erkennen läßt. Der längliche, waagerechte Abschnitt 10 des Ohrtasters 8 verläuft von der Scheibe 60 aus nach hinten, d.h. nach rechts in Fig. 3, wobei er zwischen den beiden Schenkeln 56 und 58 hindurch verläuft.

Aufgrund der vorstehend beschriebenen Ausbildung ist der Ohrtaster 8 lösbar mit dem weiteren Kopplungsglied 36 verbunden. Er kann von rechts in Fig. 2 bzw. von unten in Fig. 3 auf das weitere Kopplungsglied 36 geschoben werden. Dabei tritt der Rand der Scheibe 50 in die Ringnut 64 ein, während gleichzeitig die Kugel 54 in die Ausnehmung 62 einrastet. Die Scheibe 50 und die Ringnut 64 bilden zusammen ein einachsiges Gelenk mit der Gelenkachse g, um die der Ohrtaster 8 relativ zum weiteren Kopplungsglied 36 drehbar ist. Der längliche Abschnitt 10 hat im Bereich zwischen den beiden Schenkeln 56 und 58 eine solche Abmessung, daß die vorstehend genannte Drehung im erforderlichen Bereich möglich ist, bevor der Abschnitt 10 gegen den Schenkel 56 oder den Schenkel 56 stößt. Diejenigen Elemente, die das Gelenk zwischen dem Ohrtaster 8 und dem weiteren Kopplungsglied 36 bilden, nämlich die Scheibe 50 und die Ringnut 64, bilden zugleich eine Verbindungsvorrichtung zwischen dem Ohrtaster 8 und dem weiteren Kopplungsglied 36, wobei diese Verbindung lösbar gesichert ist durch Sicherungsmittel, die beim dargestellten Ausführungsbeispiel durch die Kugel 54 und die Ausnehmung 62 gebildet sind.

Aufgrund der vorstehend beschriebenen Geometrie des weiteren Kopplungsgliedes 36 ist dafür gesorgt, daß der Ohrtaster 8 zur Innenseite, d.h. nach links in Fig. 2, von der Parallelführungsvorrichtung vorsteht, so daß letztere das Anlegen des Ohrtasters 8 an den Kopf 4 nicht behindert und die Marken 14 von vorn, d.h. von links in Fig. 1, unbehindert sichtbar sind.

An einem der ersten Lenker, und zwar beim dargestellten Ausführungsbeispiel am ersten Lenker 22, ist ein Arm 66 befestigt, an dem quer zur Längsachse des ersten Lenkers 22 verschiebbar ein Gewichtsteil 68 befestigt ist. Ferner ist am ersten Lenker 22 ein Gegengewicht 70 befestigt. Das Gegengewicht 70 und das Gewichtsteil 68 dienen dazu, den Massenschwerpunkt der bewegbaren Elemente der ersten Parallelogrammführung 16 einschließlich des bewegbaren Kopplungsgliedes 30 derart festzulegen, daß die Gewichtskraft der ersten Parallelogrammführung 16 ein Moment um das obere Gelenk des ersten Lenkers 22 bewirkt, das seinen Größtwert hat, wenn die beiden ersten Lenker 20 und 22 senkrecht nach unten verlaufen. Dies heißt mit anderen Worten, daß durch das verschiebbare Gewichtsteil 68 und das Gegengewicht 70 der genannte Schwerpunkt S an eine Stelle gebracht wird, die auf einer Achse h liegt, die senkrecht zur Längsachse des ersten Lenkers 22 verläuft und durch dessen oberes Gelenk am vorrichtungsfesten Kopplungsglied 24 verläuft. Der Ort des Schwerpunktes S auf der letztbeschriebenen Achse h kann durch Verschieben des Gewichtsteils 68 verändert werden. Auf jeden Fall liegt er jedoch auf der dem Ohrtaster 8 zugewandten Seite des oberen Gelenks des ersten Lenkers 22. Durch diese Lage des Schwerpunktes S ist dafür gesorgt, daß die erste Parallelogrammführung 16 aufgrund ihres Gewichtes eine horizontale Zugkraft auf die zweite Parallelogrammführung 18 und somit den Ohrtaster 8 ausübt, die davon unabhängig ist, wie weit die beiden ersten Lenker 20 und 22 aus ihrer senkrechten Lage ausgelenkt sind. Dies heißt mit anderen Worten, daß diese horizontale Zugkraft konstant ist und in ihrer Größe durch die Lage des Schwerpunktes S auf der Achse h bestimmt ist. Dies ist insofern vorteilhaft, als dadurch für annähernd konstante Anlagekraft des Anlageabschnitts 12 des Ohrtasters 8 am Ohr gesorgt ist, und zwar unabhängig davon, wie weit der Ohrtaster 8 aus der Vorrichtung 2 herausgezogen ist. Dadurch erübrigen sich aufwendige Federanordnungen zum gleichen Zweck.

## Patentansprüche

1. Parallelführungseinrichtung für einen Ohrtaster (8), der einen länglichen Abschnitt (10) sowie einen Anlageabschnitt (12) aufweist, mit einer ersten Parallelogrammführung (16) mit vier Gelenken (26, 28), die zwei parallel verlaufende, stabförmige erste Lenker (20, 22), ein einrichtungsfesters Kopplungsglied (24) und ein bewegbares Kopplungsglied (30) aufweist, wobei die zwei ersten Lenker (20, 22) am bewegbaren Kopplungsglied (30) mittels einachsiger Gelenke angelenkt sind, und einer zweiten Parallelogrammführung (18), die zwei am bewegbaren Kopplungsglied (30) schwenkbar angelenkte stabförmige zweite Lenker (32, 34) sowie ein weiteres Kopplungsglied (36) aufweist, an dem der Ohrtaster (8) angebracht ist, dadurch gekennzeichnet, daß alle vier Gelenke (26, 28) der ersten Parallelogrammführung (16) als einachsige Gelenke ausgebildet sind, daß jeder der zweiten Lenker (32, 34) am bewegbaren Kopplungsglied (30) derart angelenkt ist, daß er um zwei zueinander senkrechte Gelenkachsen (c, e; d, f) schwenkbar ist, wobei die einen Gelenkachsen (c, d) der zweiten Lenker (32, 34) miteinander fluchten und die anderen Gelenkachsen (e, f) der zweiten Lenker (32, 34) parallel zueinander verlaufen, und daß der Ohrtaster (8) am weiteren Kopplungsglied (36) mittels eines einachsigen Gelenks (50, 60, 64) drehbar gelagert ist, dessen Gelenkachse (g) im wesentlichen in Richtung der Längsachse des länglichen Abschnitts (10) des Ohrtasters (8) verläuft.

2. Parallelführungseinrichtung nach Anspruch 1, dadurch gekennzeichnet, daß die Bewegungsebene der ersten Lenker (20, 22) im wesentlichen vertikal verläuft, daß die Bewegungsebene der zweiten Lenker (32, 34) um die anderen Gelenkachsen (e, f) im wesentlichen vertikal verläuft und daß diese Bewegungsebenen im wesentlichen parallel zueinander oder sich deckend verlaufen.

3. Parallelführungseinrichtung nach Anspruch 1 oder 2, dadurch gekennzeichnet, daß jeder zweite Lenker (32, 34) am bewegbaren Kopplungsglied (30) mittels eines Kreuzgelenks gelagert ist.

4. Parallelführungseinrichtung nach Anspruch 1 oder 2, gekennzeichnet durch ein am bewegbaren Kopplungsglied (30) um eine Achse (c-d) drehbar gelagertes Teil (38), an dem die beiden zweiten Lenker (32, 34) jeweils mittels eines einachsigen Gelenks (44, 46) gelagert sind.

5. Parallelführungseinrichtung nach einem der Ansprüche 1 bis 4, dadurch gekennzeichnet, daß die zweiten Lenker (32, 34) mit dem weiteren Kopplungsglied (36) jeweils mittels eines Kugelgelenks verbunden sind.

6. Parallelführungseinrichtung nach einem der Ansprüche 1 bis 5, dadurch gekennzeichnet, daß der Ohrtaster (8) am weiteren Kopplungsglied (36) lösbar mittels einer Verbindungsvorrichtung (50, 60, 64) angebracht ist.

7. Parallelführungseinrichtung nach Anspruch 6, dadurch gekennzeichnet, daß das einachsige Gelenk (50, 60, 64) zwischen dem Ohrtaster (8) und dem weiteren Kopplungsglied (36) zugleich die lösbare Verbindungsvorrichtung (50, 60, 64) bildet.

8. Parallelführungseinrichtung nach Anspruch 7, dadurch gekennzeichnet, daß das Gelenk (50, 60, 64) zwischen dem Ohrtaster (8) und dem weiteren Kopplungsglied (36) eine am weiteren Kopplungsglied angeordnete, kreiszylindrische Scheibe (50), deren Zylinderachse mit der Gelenkachse (g) des Gelenks zwischen dem Ohrtaster und dem weiteren Kopplungsglied zusammenfällt, und eine den Rand der Scheibe lediglich entlang eines Abschnitts ihres Umfangs umgreifende Ringnut (64) am Ohrtaster umfaßt, in der die Scheibe drehbar ist, und daß Sicherungsmittel (54, 62) die Scheibe und die Ringnut in ihrer Eingriffsstellung sichern.

9. Parallelogrammführungseinrichtung nach Anspruch 8, dadurch gekennzeichnet, daß die Sicherungsmittel eine federbelastete Kugel (54) umfassen, die auf der Gelenkachse (g) angeordnet ist, in der Scheibe (50) oder dem Ohrtaster (8)gehalten ist und in eine Ausnehmung (62) am jeweils anderen Element (Ohrtaster oder Scheibe) eingreift.

10. Parallelführungseinrichtung nach einem der Ansprüche 1 bis 9, dadurch gekennzeichnet, daß der Massenschwerpunkt (S) der bewegbaren Elemente der ersten Parallelogrammführung (16) einschließlich ihres bewegbaren Kopplungsgliedes (30) auf einer Achse (h) angeordnet ist, die senkrecht zur Längsachse eines der ersten Lenker (20, 22) und durch dessen Gelenk am vorrichtungsfesten Kopplungsglied (24) verläuft, wobei der Massenschwerpunkt auf der dem Ohrtaster (8) zugewandten Seite dieses Gelenks liegt.

11. Parallelogrammführungseinrichtung nach Anspruch 10, gekennzeichnet durch einen an einem der ersten Lenker (20, 22) befestigten Arm (66) und ein auf diesem verschiebbares Gewichtsteil (68) zur Einstellung des Ortes des Massenschwerpunktes (S).

## Claims

1. A parallel guiding device for an ear sensor (8) which has an elongate portion (10) and a contact portion (12), said guiding device comprising a first parallelogram guide (16) having four articulations (26, 28), two parallel, rod-shaped first links (20, 22), a coupling member (24) rigidly connected to the device and a movable coupling member (30), with the two first links (20, 22) being articulated to the movable coupling member (30) by means of single-axis articulations, and a second parallelogram guide (18) having two rod-shaped second links (32, 34) pivotally mounted on the movable coupling member (30), and a further coupling member (36) on which the ear sensor (8) is disposed, **characterized in that**
all four articulations (26, 28) of the first parallelogram guide (16) are single-axis articulations, that each of the second links (32, 34) is articulated to the movable coupling member (30) such that it is pivotable around two articulation axes (c, e; d, f) perpendicular to one another, with the one articulation axes (c, d) of the second links (32, 34) being in alignment with one another and the other articulation axes (e, f) of the second links (32, 34) extending parallel to one another, and that the ear sensor (8) is rotatably mounted on the further coupling member (36) by means of a single-axis articulation (50, 60, 64) whose axis (g) extends substantially in the direction of the longitudinal axis of the elongate portion (10) of the ear sensor (8).

2. A parallel guiding device according to claim 1, **characterized in that** the movement plane of the first links (20, 22) is substantially vertical, that the movement plane of the second links (32, 34) around the other articulation axes (e, f) is substantially vertical, and that these movement planes extend substantially parallel to or congruent with one another.

3. A parallel guiding device according to claim 1 or 2, **characterized in that** every second link (32, 34) is mounted on the movable coupling member (30) by means of a universal joint.

4. A parallel guiding device according to claim 1 or 2, **characterized** by a member (38) mounted on the movable coupling member (30) for rotation around an axis (c-d), the two second links (32, 34) being mounted each by means of a single-axis articulation (44, 46) on the member (38).

5. A parallel guiding device according to any of claims 1 to 4, **characterized in that** the second links (32, 34) are each connected to the further coupling member (36) by means of a ball joint.

6. A parallel guiding device according to any of claims 1 to 5, **characterized in that** the ear sensor (8) is releasably disposed on the further coupling member (36) by means of a connecting device (50, 60, 64).

7. A parallel guiding device according to claim 6, **characterized in that** the single-axis articulation (50, 60, 64) between the ear sensor (8) and the further coupling member (36) at the same time forms the releasable connecting device (50, 60, 64).

8. A parallel guiding device according to claim 7, **characterized in that** the articulation (50, 60, 64) between the ear sensor (8) and the further coupling member (36) comprises a cylindrical disc (50) disposed on the further coupling member (36), the cylinder axis of the disc (50) coinciding with the articulation axis (g) of the articulation between the ear sensor (8) and the further coupling member, and an annular groove (64) formed at the ear sensor (8) and extending around the disc edge along only a part of its periphery, the dic being rotatable in the groove (64), and that securing means (54, 62) secure the disc and the annular groove in their engaged position.

9. A parallel guiding device according to claim 8, **characterized in that** the securing means comprise a spring-loaded ball (54) which is disposed on the articulation axis (g), is retained in the disc (50) or the ear sensor (8), and engages in a recess (62) in the respective other element (ear sensor or disc).

10. A parallel guiding device according to any of claims 1 to 9, **characterized in that** the centre of gravity (S) of the movable elements of the first parallelogram guide (16) including its movable coupling member (30) is disposed on an axis (h) which extends perpendicularly to the longitudinal axis of one of the first links (20, 22) and through its articulation at the coupling member (24) rigidly secured to the device, the centre of gravity being disposed on that side of the latter articulation which faces the ear sensor (8).

11. A parallel guiding device according to claim 10, **characterized** by an arm (66) secured to one of the first links (20, 22) and a weight (68) movable on the arm (66) and adapted to adjust the position of the centre of gravity (S).

## Revendications

1. Dispositif de guidage parallèle d'un palpeur d'oreille (8) qui comporte une partie allongée (10) ainsi qu'une partie d'appui (12), ledit dispositif comprenant un premier guidage à parallélogramme (16) qui possède quatre articulations (26, 28) et qui comporte deux premiers bras parallèles en forme de barres (20, 22), un élément d'accouplement (24) solidaire du dispositif et un élément d'accouplement mobile (30), les deux premiers bras (20, 22) étant articulés sur l'élément d'accouplement mobile (30) au moyen d'articulations uniaxes, et comprenant un second guidage à parallélogramme (18) qui comporte deux seconds bras en forme de barres (32, 34) articulés de manière pivotante sur l'élément d'accouplement mobile (30) ainsi qu'un élément d'accouplement supplémentaire (36) sur lequel est monté le palpeur d'oreille (8), caractérisé en ce que les quatre articulations (26, 28) du premier guidage à parallélogramme (16) sont toutes conçues sous la forme d'articulations uniaxes, en ce que chacun des seconds bras (32, 34) est articulé sur l'élément d'accouplement mobile (30) de façon à pouvoir pivoter selon deux axes d'articulation (c, e ; d, f) perpendiculaires l'un à l'autre, les premiers axes d'articulation (c, d) des seconds bras (32, 34) étant dans l'alignement l'un de l'autre, et les seconds axes d'articulation (e, f) des seconds bras (32, 34) étant parallèles l'un à l'autre, et en ce que le palpeur d'oreille (8) est monté tournant sur l'élément d'accouplement supplémentaire (36) au moyen d'une articulation uniaxe (50, 60, 64) dont l'axe d'articulation (g) s'étend sensiblement dans la direction de l'axe longitudinal de la partie allongée (10) du palpeur d'oreille (8).

2. Dispositif de guidage parallèle selon la revendication 1, caractérisé en ce que le plan de déplacement des premiers bras (20, 22) est sensiblement vertical, en ce que le plan de déplacement des seconds bras (32, 34) selon les seconds axes d'articulation (e, f) est sensiblement vertical, et en ce que ces plans de déplacement sont sensiblement parallèles l'un à l'autre ou coïncident l'un avec l'autre.

3. Dispositif de guidage parallèle selon la revendication 1 ou 2, caractérisé en ce que chacun des seconds bras (32, 34) est monté sur l'élément d'accouplement mobile (30) au moyen d'une articulation à croisillon.

4. Dispositif de guidage parallèle selon la revendication 1 ou 2, caractérisé en ce qu'il comprend une pièce (38) qui est montée tournante sur l'élément d'accouplement mobile (30) selon un axe (c-d) et sur laquelle chacun des deux seconds bras (32, 34) est monté au moyen d'une articulation uniaxe (44, 46).

5. Dispositif de guidage parallèle selon l'une des revendications 1 à 4, caractérisé en ce que chacun des seconds bras (32, 34) est relié à l'élément d'accouplement supplémentaire (36) au moyen d'une articulation à croisillon.

6. Dispositif de guidage parallèle selon l'une des revendications 1 à 5, caractérisé en ce que le palpeur d'oreille (8) est monté de manière détachable sur l'élément d'accouplement supplémentaire (36) au moyen d'un dispositif de liaison (50, 60, 64).

7. Dispositif de guidage parallèle selon la revendication 6, caractérisé en ce que l'articulation uniaxe (50, 60, 64) entre le palpeur d'oreille (8) et l'élément d'accouplement supplémentaire (36) constitue en même temps le dispositif de liaison détachable (50, 60, 64).

8. Dispositif de guidage parallèle selon la revendication 7, caractérisé en ce que l'articulation (50, 60, 64) entre le palpeur d'oreille (8) et l' élément d'accouplement supplémentaire (36) comprend un disque cylindrique circulaire (50), qui est monté sur l'élément d'accouplement supplémentaire et dont l'axe du cylindre coïncide avec l'axe d'articulation (g) de l'articulation entre le palpeur d'oreille et l'élément d'accouplement supplémentaire, et, sur le palpeur d'oreille, une gorge annulaire (64) qui n'entoure le bord du disque que sur une partie de sa périphérie et dans laquelle le disque peut tourner, et en ce que des moyens de blocage (54, 62) bloquent le disque et la gorge annulaire en position emboîtée.

9. Dispositif de guidage à parallélogramme selon la revendication 8, caractérisé en ce que les moyens de blocage comprennent une bille à sollicitation élastique (54) qui est montée sur l'axe d'articulation (g), est maintenue dans le disque (50) ou dans le palpeur d'oreille (8) et pénètre dans un évidement (62) pratiqué dans l'autre élément (palpeur d'oreille ou disque).

10. Dispositif de guidage parallèle selon l'une des revendications 1 à 9, caractérisé en ce que le centre de gravité (S) des éléments mobiles du premier guidage à parallélogramme (16), y compris de son élément d'accouplement mobile (30), se trouve sur un axe (h) qui est perpendiculaire à l'axe longitudinal de l'un des premiers bras (20, 22) et qui passe par l'articulation dudit premier bras sur l'élément d'accouplement (24) solidaire du dispositif, le centre de gravité se trouvant sur le côté de cette articulation tourné vers le palpeur d'oreille (8).

11. Dispositif de guidage à parallélogramme selon la revendication 10, caractérisé en ce qu'il comprend une tige (66) fixée à l'un des premiers bras (20, 22) et une pièce formant poids (68) qui peut se déplacer sur ladite tige et qui sert à situer le centre de gravité (S).
